# EUROPEAN PATENT APPLICATION

(11) **EP 4 354 120 A1**
(43) Date of publication of application: **17.04.2024**
(21) Application number: 22382986.2
(22) Date of filing: 14.10.2022
(51) Int. Cl.: G01N 21/78, B01L 3/00, G01N 31/22, G01N 33/14, G01N 21/77

(54) **MICROFLUIDIC DEVICE FOR THE DETECTION OF DRUGS IN BEVERAGES**

(71) Applicant: Alba Biotech, S.L., 01005 Vitoria-Gasteiz (Alava) (ES); Universidad del País Vasco/Euskal Herriko Unibertsitatea, 48940 Leioa, Vizcaya (ES)
(72) Inventor: Basabe Desmonts, Lourdes, 48940 LEIOA (BIZKAIA) (ES); Benito López, Fernando, 48940 LEIOA (BIZKAIA) (ES); Azuaje Hualde, Enrique, 48940 LEIOA (BIZKAIA) (ES); Álvarez Braña, Yara, 48940 LEIOA (BIZKAIA) (ES); Poves Ruiz, Isabel, 01005 Vitoria-Gasteiz (Alava) (ES); Carasa Oyarzabal, Fernando, 01005 Vitoria-Gasteiz (Alava) (ES); Colás Fernández, Jose Javier, 01005 Vitoria-Gasteiz (Alava) (ES); Silgo Hernández, Jose Miguel, 01005 Vitoria-Gasteiz (Alava) (ES); Fernández de Arbina Urbina, Ane, 01005 Vitoria-Gasteiz (Alava) (ES)
(74) Representative: Isern Patentes y Marcas S.L.

(57) **Abstract**

The present invention provides a device for the detection of compounds. Particularly, the present invention relates to a microfluidic device for the detection of rape drugs based on the colorimetric analysis thereof, as well as the method for use thereof.

## Description

### STATE OF THE ART

Sexual assault and sexual abuse are among the most common crimes in the world today. Drug facilitated sexual assault (DFSA) occurs when a person is subdued while incapacitated due to the effects of alcohol or drugs, commonly known as date rape drugs. These drugs have become very popular among abusers, since they not only physically incapacitate the victim, but they also hamper their mental capacity so that they cannot remember anything.

The most common date rape drugs are central nervous system depressants, leading to pharmacological effects such as relaxation, lack of inhibition, amnesia, altered perception, drowsiness and unconsciousness, among others. These drugs produce fast-acting, short-lasting effects at low doses and can be easily mixed into foods and beverages, since they do not have an odour, flavour or colour. Substances most commonly used as date rape drugs include hypnotics and benzodiazepines, analgesics-anaesthetics (for example, ketamine or fentanyl), scopolamine and γ-hydroxybutyric acid (GHB).

For example, ketamine is an anaesthetic drug used for medical purposes, which causes memory loss, visual hallucinations and distortion of time and space. Furthermore, benzodiazepines comprise a family of central nervous system depressants that include diazepam, midazolam, clonazepam and flunitrazepam, among others. They have anxiolytic, sedative and hypnotic properties, which increase when the drug is taken in combination with alcohol.

Commonly, the detection of these drugs in cases of sexual assault is carried out using highly sophisticated instrumental techniques such as liquid or gas chromatography coupled with mass spectrometry. Despite the high level of sensitivity, accuracy and specificity of chromatographic methods, they are not suitable for *on-site* analysis due to the complexity of the instrumentation and the requirement for strict laboratory conditions and trained personnel.

Various strategies have been used, including electrochemical and colour-based detection methods, for *on-site* test generation for the detection of date rape drugs. Particularly, colour-based tests have been used for decades in preemptive *on-site analysis* of samples due to the simplicity, speed and portability thereof. Advances in fields such as digital colour analysis, the development of solid colorimetric sensors and microfluidic devices have led to a growing interest in the use of these types of tests at the point of use.

There are commercially available colour-based tests to detect date rape drugs in beverages, such as Drink Guard and Drink Detective. The Drink Guard test detects Ketamine and GHB, and the Drink Detective can detect many more drugs such as Ketamine, benzodiazepines or GHB. However, these tests were shown to give false positives and, in addition, they show problems for the detection of drugs in different matrices.

In recent years, paper-based microfluidic analytical devices have emerged, since they are simple *on-site* colorimetric tests for a multiple analysis of a wide variety of analytes. These systems are based on simple chemical reactions that produce visible results that can be interpreted with the naked eye. In recent years, several microfluidic devices have been developed for the naked eye detection of psychotropic compounds, including ketamine, using colorimetric reactions. For example, Musile et al. developed a six-channel paper-based microfluidic device for naked-eye detection of ketamine, among other psychotropic drugs, including morphine, epinephrine and codeine. More recently, Yehia et al. developed a trimodal system in a paper-based microfluidic device that combines potentiometric, fluorometric and colorimetric sensors for the sensitive and specific detection of ketamine. However, in both cases the colorimetric reaction presented a high detection limit, disabling its use in a real case scenario.

### SUMMARY OF THE INVENTION

The present invention provides a device for the detection of compounds. Particularly, the present invention relates to a microfluidic device for the detection of rape drugs based on the colorimetric analysis thereof, as well as the method for use thereof.

### LIST OF FIGURES

Figure 1: Structure of the microfluidic device.
Figure 2: Covered and uncovered microfluidic device.

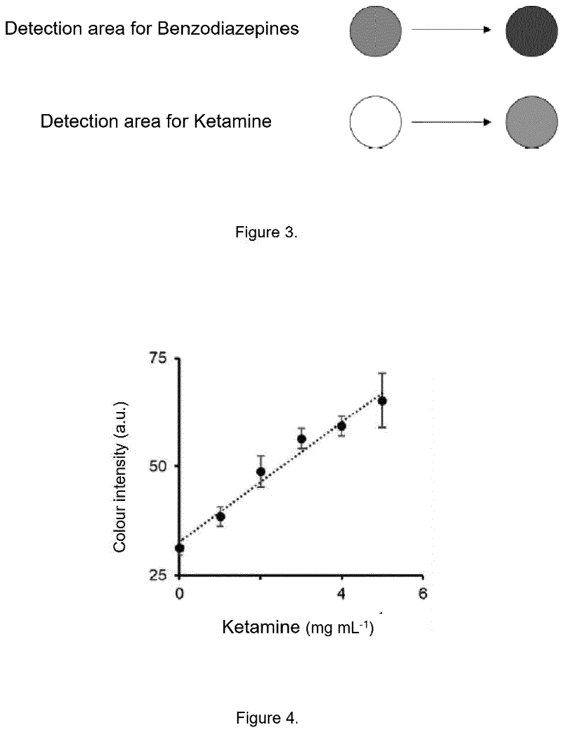

Figure 3. Colour changes due to benzodiazepines and ketamine (Morris and Scott reagent).
Figure 4. Calibration curve for the detection of ketamine.

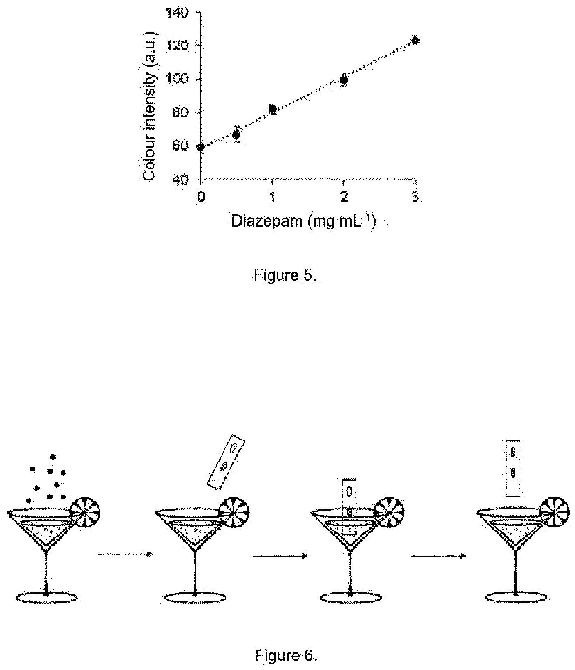

Figure 5. Calibration curve for the detection of benzodiazepines.
Figure 6: Method for detecting the presence of date rape drugs in beverages.

### DETAILED DESCRIPTION

The present invention provides a microfluidic device for the detection of rape drugs in beverages based on the colorimetric analysis thereof.

In a preferred embodiment, the present invention relates to microfluidic devices comprising several overlapping layers. Preferably, the microfluidic devices comprise an overlapping of bottom, middle, top layers and an intermediate detection layer comprising a microfluidic structure. Preferably, the overlapping layers form a rectangular device.

Preferably, the bottom layer comprises a hydrophilic layer of a pressure sensitive adhesive (PSA). Preferably the pressure sensitive adhesive (PSA) is a hydrophilic material with a contact angle between 6° - 7°. Preferably, the PSA comprises a polyester film coated on both sides with acrylic adhesive, for example, ARflow^{®}93049.

Preferably, the intermediate detection layer comprises a microfluidic structure, wherein the microfluidic structure comprises an opening, a channel, at least two detection areas and one output channel, arranged sequentially, wherein the intermediate layer comprises a double-sided PSA. Preferably the pressure sensitive adhesive (PSA) is a hydrophilic material with a contact angle between 6° - 7°. Preferably, the PSA comprises a polyester film coated on both sides with acrylic adhesive, for example, ARflow^{®}93049. The intermediate detection layer allows a sufficient volume of beverage sample to flow from the beverage to the detection area due to the effect of capillarity. It also provides the required distance for the liquid to flow upwards and create the space to hold the required sample volume.

Preferably, the microfluidic structure comprises a purge channel arranged before the detection chamber.

Preferably, the detection layer comprises the necessary reagents for the identification reaction on a cellulose material. As defined here, the cellulose material is selected from nitrocellulose, carboxymethylcellulose, diethylaminoethylcellulose, phosphocellulose, quaternary ammonium substituted cellulose and sulfoxyethylcellulose. Preferably, the reagents are impregnated with nitrocellulose. Preferably, the reagents can be found in ionogels on cellulose materials or any similar material.

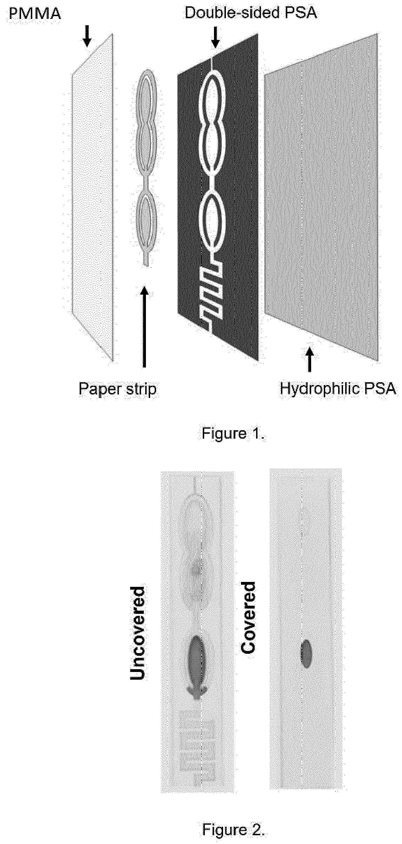

Preferably, the detection layer comprises two partially closed sections which contain the detection areas joined by a branched channel that starts from the inlet channel forming two channels symmetrical with respect to the longitudinal axis that surround the first partially closed section to join the channel that joins the two partially closed segments, wherein the channel that joins the two partially closed sections bifurcates, forming two channels symmetrical with respect to the longitudinal axis of the same so that they surround the second partially closed section and join to form the outlet channel. The arrangement of the detection layer is described in Figure 1.

Preferably, the cellulose material comprises two partially closed sections joined by a branched channel, wherein the branched channel starts from the inlet channel forming two channels symmetrical with respect to the longitudinal axis that surround the first partially closed section to join the channel that joins the two partially closed sections, wherein the channel that joins the two partially closed sections bifurcates, forming two channels symmetrical with respect to the longitudinal axis of the same so that they surround the second partially closed section and join to form the outlet channel. Preferably, the second partially closed section is made up of two partially overlapping circles. The arrangement of the cellulose material is described in Figure 1.

The configuration of the cellulose material allows the passage of the liquid towards the first partially closed section wherein benzodiazepine is detected and simultaneously the passage of the liquid towards the second partially closed section where ketamine is detected. The configuration in two ellipses of the second partially closed section allows the reaction of ketamine with the colorimetric compound in the first ellipse and the subsequent accumulation of the resulting colorimetric compound in the second ellipse due to the effect of capillarity.

The top layer comprises an opening or window overlapping the detection area so that the colour change can be seen with the naked eye. The top layer can be a layer of functionalised hydrophilic or hydrophobic material, such as polymethylmethacrylate (PMMA), polymethacrylate (PMA), polyethylene terephthalate (PET), polydimethylsiloxane (PDMS), thermoplastic elastomer (TPE), polystyrene (PS), polycarbonate (PC), polyethylene glycol diacrylate (PEGDA), polyethylene glycol (PEG), polyurethane (PU), cyclic olefin copolymer (COC), cyclic olefin polymer (COP), teflons, hydrogels and ionogels. Preferably, the top layer is made of polymethylmethacrylate (PMMA).

In a preferred embodiment, the detection area comprises a lower, middle and upper circle sequentially joined on a longitudinal axis by a channel. Preferably, the detection area comprises a circle from which a channel protrudes.

In a preferred embodiment, the bottom layer comprises a thickness between 100 and 500 µm, the middle layer comprises a thickness between 50 µm and 1 mm, and the top layer comprises a thickness between 100 and 500 µm.

In a preferred embodiment, the length of the cellulose material comprises a length between 1 and 10 cm, preferably 5 cm. The length of the device allows enough colour to accumulate for observation.

The detection layer has the required reagents to generate a colour change in the presence of chemical markers (inorganic molecules, metals and ions, such as Na⁺, K⁺, Li⁺, I⁻, Cl⁻, nitrates, nitrites, Cu, Co, and Fe ions, H₂O, O₂, CO₂) and biochemicals (organic molecules, metabolic derivatives, large molecules, such as ethanol, organometallic compounds, glucose, lactate, disaccharides, lipids, proteins, lipoproteins, nucleic acids, DNA, hormones).

In a preferred embodiment, the intermediate detection layer can detect the presence of "date rape drugs" after receiving the sample to be tested. The detection layer has the required reagents to generate a colour change. As defined here, "date rape drugs" preferably comprise benzodiazepines and ketamine, with drugs such as scopolamine and gamma hydroxybutyric acid (GHB) among others being able to be included in this group. As defined here, benzodiazepines comprise any of the following compounds: diazepam, midazolam, clonazepam, flunitrazepam, temazepam, stalozolam, alprazolam, lormetazepam, lorazepam, bromazepam, nitrazepam, chlordiazepoxide, albandazole, meloxicam.

For the detection of benzodiazepines, colorimetric or luminescent reactions, preferably, but not limited to, the Scott reaction, are used, titration-type reactions, immunoassays, competitive immunoassay, enzyme-mediated immunoassay, enzymatic reactions, and aptamers among others being able to be used. The Scott reaction consists of the formation of a colour complex between benzodiazepine and cobalt (II) thiocyanate in an acid medium. According to the reaction, a colour change to pink for the negative result and to blue / purple for the positive result is observed.

For the detection of ketamine, colorimetric or luminescent reactions, preferably, but not limited to, the Morris's reaction, are used; titration-type reactions, immunoassays, competitive immunoassay, enzyme-mediated immunoassay, enzymatic reactions, and aptamers among others being able to be used. The Morris reaction consists of the formation of a colour complex between ketamine and cobalt (II) thiocyanate in a basic medium. According to this reaction, no colour change was observed for the negative result and a blue colour for the positive result.

For the detection of other rape drugs such as scopolamine and GHB, colorimetric or luminescent reactions are used, as well as titration-type reactions, immunoassays, competitive immunoassay, enzyme mediated immunoassay, enzymatic reactions, and aptamers among others.

In a preferred embodiment, the method for detecting the presence of rape drugs in beverages comprises inserting the microfluidic device into the beverage for a few seconds comprised between 1 and 10 seconds, preferably 5 seconds, and filling the sample deposit of the device. Waiting for at least 1 minute, preferably 3 minutes, more preferably 5 minutes and evaluate the colour of the device, preferably with the naked eye or by means of electronic means.

Multi-layer configuration guarantees the protection of the sensor, prevents leaking of reagents into the beverage and acts as a deposit to store the exact volume of liquid required to wet all of the cellulose material and ensure the autonomous performance of the device.

In a preferred embodiment, the device can be laminated to provide protection from the external environment without compromising test integrity by allowing sample permeability during use. Preferably, the apparatus can be waterproof, or substantially waterproof, until the device is activated, for example, after removal of a removable layer or other methods.

### EXAMPLES

### Ketamine detection (Example 1)

For the assembly of the detection area, 25 µl of the ketamine sensor solution were placed on the lower circle of the paper and allowed to dry. The ketamine sensor consisted of a suspended precipitate, so that when the solution was deposited on the paper, a green precipitate can be observed in the lower circle, with a light pink supernatant that can be seen in the lower half of the cellulose material. This precipitate reacts to form the complex, becoming soluble.

For the detection of ketamine in beverages, the end of the device, which comprises the inlet, was immersed in the beverage for 5 s to fill it and then withdrawn from the beverage. Then, the stored liquid flowed autonomously through the paper to the end point of the cellulose material. When the adulterated beverage passed through the detection area, a colour change could be observed both in the lower circle, where the sensor solution was deposited, and on the upper area of the paper cellulose material, including the middle and upper circles. The device introduced in a solution of 5 mg of ketamine ml-1 showed two colour changes. First, the precipitate located in the lower circle turned into a bluish-purple colour with time. The supernatant, which flowed from the detection area to the end point of the cellulose material, turned into a bright blue colour, noticeable in the upper circle 5 minutes after charging the device, but immediately after the solution reached the sensor. When introducing the device into unadulterated beverages (for example, distilled water), the colour of the precipitate formed in the first circle remained green, and the supernatant that can be observed in the upper area of the device turns into a light pink colour.

### Sensitivity for ketamine (Example 2)

To test the sensitivity of the device, a calibration curve was carried out for the ketamine microfluidic device. For this purpose, 6 ketamine solutions were prepared, including five different concentrations of the drug: 0, 1, 2, 3, 4, 5 mg ml-1, (n = 3). Images were obtained on the devices 10 minutes after loading the solutions. It can be observed that the blue colour is observable from the middle to the end point of the cellulose material, showing positive results. Ketamine microfluidic device P hotographs were analysed and processed by ImageJ. All images were transformed into RGB images. Red values were chosen for the calibration curve. The calibration curve equation was y = 6.86x + 32.75 (R2: 0.9740), obtaining a detection limit (LoD) of 0.71 mg ml-1.

Taking into account the effective oral dose of ketamine in its anaesthetic use (6-10 mg kg-1), and the possible volume of the different vessels (30 - 500 ml), the concentration of ketamine that can be found in adulterated beverages varies from 0.72 mg ml-1 to 20 mg ml-1. In its current form, the ketamine microfluidic device allows proper detection of ketamine for all possible concentrations found in real life settings.

To test the device on different matrices, a variety of conventional beverages were chosen including Coca-Cola, Orange Kas, Lemon Kas, Schweppes Tonic and Gin (Steward) + Schweppes Tonic (3: 7). A 5 mg ml-1 ketamine solution was prepared with each matrix. A 5 mg ml-1 ketamine solution was prepared with each matrix.

A ketamine-adulterated beverage produced a colour change to blue in the upper middle section of the cellulose material. However, negative results showed unexpected colourings depending on the type of matrix. For the Lemon Kas and Gin-Tonic matrices, the devices had a distinctive colour change to light pink, whereas with other beverages, such as Coca-Cola, Orange Kas and Tonic, negative devices had a slightly blue colouring near the end point. However, a large difference was still observed between the colourings of the negative and positive devices. These results indicate that the ketamine microfluidic device is capable of detecting adulteration in a wide range of common beverages. Curiously, the colouring of the detection area of the band where the precipitate was deposited (lower circle) tended to disappear with the flow of the beverages. This indicates that the pH and composition of the beverage affect not only the detection reaction, but also the ability to dissolve the precipitate. In addition, this confirms the need to place the window between the middle and upper circle to avoid false positive results.

### Benzodiazepine detection (Example 3)

For the assembly of the detection area, 9.5 µl of the benzodiazepine sensor solution were placed on the single circle on the paper and allowed to dry. In the case of benzodiazepines, the sensor was a homogeneous solution with no appreciable precipitate, resulting in a homogeneous colour throughout the paper detection area. Its operating principle is similar to that of ketamine and consisted of inserting the end of the device, where the inlet d is placed, in the beverage for 3 s to fill the channel deposit with the sample liquid. Once full, the microfluidic device was withdrawn from the beverage. Immediately after, the stored liquid flowed towards the end point of the cellulose material absorbing the sample and wetting the material along the way.

After evaporation of the sensor solution, the colour of the cellulose material was found to be blue. When introduced into unadulterated beverages (for example, distilled water), the colour changes from blue to light pink. Furthermore, in the case of a positive result, when the benzodiazepine microfluidic device is introduced into a 2.5 mg ml-1 diazepam solution, a colour change from blue to purple was already observed during the first minute of operation.

### Sensitivity to benzodiazepines (Example 4)

5 diazepam solutions were prepared and tested with the device, including 0, 0.5, 1, 2 and 3 mg ml-1 of diazepam in distilled water. Photographs of the benzodiazepine microfluidic device were analysed and processed by ImageJ using the same protocol as that used for ketamine. The calibration curve equation was: y = 21.47x + 58.37 (R2: 0.9947), with 0.55 mg ml-1 as detection limit.

Considering the effective oral dose for diazepam when used as a hypnotic (10-20 mg), and the possible volume of the different vessels (30 - 500 ml), the concentration of benzodiazepines that can be found in adulterated beverages varies from 0.02 mg ml-1 to 0.66 mg ml-1. In its current form, the detection limit of the benzodiazepine microfluidic device is within the range of diazepam concentrations that can be found in real life settings.

To test different matrices, the same conventional beverages were used as for the ketamine microfluidic device. The matrices were adulterated with 2.5 mg ml-1 diazepam.

When introduced into unadulterated beverages, the colour changed from blue to light pink. In all cases, when the device was introduced into an adulterated beverage, the colour changed to purple.

### Combined ketamine and benzodiazepine microfluidic device (Example 5)

The assembly method was the same as in previous sections where 6.5 µl of the benzodiazepine sensor solution were placed in the lower circle, while 7.5 µl of the ketamine sensor solution were placed in the middle circle and allowed to dry. The microfluidic device was manually aligned and assembled from the bottom layers to the top layers.

This microfluidic device used the same operating principle as its previous individual parts, wherein the inlet channel was immersed in the beverage for 5 s to fill the channel of the deposit. In this case, the liquid passed through both sensors while wetting the cellulose material, first through the benzodiazepine sensor and then through the ketamine sensor. The benzodiazepine sensor is a homogeneous solution that does not require a fluidic path. Contrary to that, the ketamine sensor forms a precipitate that dissolves after reaction with the drug, so a paper path is required to accumulate the blue colour. This accumulation occurs at the end of the central circle, so the viewing window was placed over an upper circle. The upper circle was also required to move the sample over the detection area for the required time and, therefore, to observe both drugs through colourimetry.

The colours before the device was introduced in the beverage were: blue for the benzodiazepine sensor and green for the ketamine sensor precipitate. When the inlet of the microfluidic device was immersed in the distilled water as a negative control, the benzodiazepine sensor changed colour to pink, the ketamine precipitate changed colour to light green and the ketamine supernatant observed in the upper-middle portion of the ketamine paper device turned into a light pink, as shown above.

For the detection of benzodiazepines, the combined microfluidic device was introduced into a 2.5 mg ml-1 diazepam solution (n = 3). As expected, the benzodiazepine sensor changed colour from blue to purple in the first 5 minutes. In this case, the interaction between diazepam and the ketamine edge area did not produce a change in colour, appearing similar to negative controls with unadulterated beverages.

## Claims

1. A device for detecting the presence of at least one date rape drug in beverages comprising:
- a hydrophilic bottom layer,
- at least one intermediate detection layer comprising double-sided pressure-sensitive adhesive comprising a microfluidic structure, wherein the microfluidic structure comprises an opening, a channel, at least two separate detection areas linked by a channel, and an outlet channel arranged sequentially, wherein the detection areas joined by a channel comprise a cellulose material,
- a hydrophilic top layer having at least two openings overlapping the detection areas; and wherein the cellulose material comprises a reagent that reacts with the date rape drug to form a colour complex.

2. The device according to the preceding claim, wherein the cellulose material comprises two partially closed sections joined by a branched channel, wherein the branched channel starts from the inlet channel forming two channels symmetrical with respect to the longitudinal axis that surround the first partially closed section to join the channel that joins the two partially closed sections, wherein the channel that joins the two partially closed sections bifurcates, forming two channels symmetrical with respect to the longitudinal axis of the same so that they surround the second partially closed section and join to form the outlet channel.

3. The device according to any preceding claim, wherein the channel that connects the opening and the detection areas is arranged so as to allow enough volume for the beverage to reach the detection areas due to the effect of capillarity.

4. The device according to any preceding claim, wherein the bottom layer comprises a single-sided pressure-sensitive adhesive, preferably the single-sided pressure-sensitive adhesive is a polyester film coated on one side with an acrylic adhesive.

5. The device according to any preceding claim, wherein the double-sided pressure-sensitive adhesive is a polyester film coated on both sides with an acrylic adhesive.

6. The device according to any preceding claim, wherein the cellulose material is selected from nitrocellulose, ionogels on cellulose paper, cellulose esters, carboxymethylcellulose, diethylaminoethylcellulose, phosphocellulose, quaternary ammonium substituted cellulose and sulfoxyethylcellulose.

7. The device according to any preceding claim, wherein the top layer material is selected from polymethyl methacrylate (PMMA), polymethacrylate (PMA), polyethylene terephthalate (PET), polydimethylsiloxane (PDMS), thermoplastic elastomer (TPE), polystyrene (PS), polycarbonate (PC), polyethylene glycol diacrylate (PEGDA), polyethylene glycol (PEG), polyurethane (PU), cyclic olefin copolymer (COC), cyclic olefin polymer (COP), teflons, hydrogels and ionogels.

8. The device according to any preceding claim, wherein the date rape drugs are selected from the group of benzodiazepines and/or ketamine, are preferably selected from ketamine, diazepam, midazolam, clonazepam, flunitrazepam, temazepam, stalozolam, alprazolam, lormetazepam, lorazepam, bromazepam, nitrazepam, chlordiazepoxide, albandazole, meloxicam.

9. The device according to any preceding claim, wherein the detection solution comprises the required reagents to generate a colour change in the presence of chemical and biochemical markers, preferably comprises cobalt (II) thiocyanate, preferably comprises cobalt (II) thiocyanate in acid medium or basic medium.

10. The device according to any preceding claim, wherein the microfluidic structure comprises a purge channel arranged before the detection chamber.

11. A use of a device according to any preceding claim for detecting the presence of date rape drugs in liquids, preferably benzodiazepines and/or ketamine in liquids, preferably beverages.

12. A method for detecting the presence of date rape drugs, preferably benzodiazepines and/or ketamine, in beverages comprising the steps of:
i. immersing a device according to any of the preceding claims 1-10 between 1 and 10 seconds, preferably at least 5 seconds in a beverage,
ii. extracting the device from the beverage,
iii. waiting at least 1 minute, preferably 3 minutes,
iv. evaluating the colour of the device, preferably with the naked eye.
